# FASCICULE DE BREVET EUROPEEN

(11) **EP 0 832 899 B1**
(45) Date de publication et mention de la délivrance du brevet: **17.07.2002**
(21) Numéro de dépôt: 97402232.9
(22) Date de dépôt: 25.09.1997
(51) Int. Cl.: C07H 15/04

(54) **Composition de lactitol et son procédé de préparation**
Laktitolzusammensetzung und Verfahren zu deren Herstellung
Lactitol composition and process for the preparation thereof

(30) Priorité: 27.09.1996 FR 9611804
(43) Date de publication de la demande: 01.04.1998
(73) Titulaire: Roquette Frères, 62136 Lestrem (FR)
(72) Inventeur: Caboche, Jean-Jacques, 62131 Drouvin Le Marais (FR)
(74) Mandataire: Boulinguiez, Didier

(56) Documents cités:
- EP-A- 0 039 981
- EP-A- 0 381 483
- WO-A-90/06317
- WO-A-92/16542
- PATENT ABSTRACTS OF JAPAN vol. 015, no. 001 (C-0793), 7 janvier 1991 & JP 02 255694 A (TOWA KASEI KOGYO KK), 16 octobre 1990,

## Description

La présente invention concerne une nouvelle composition cristalline de lactitol anhydre, d'une très grande pureté cristalline et de structure poreuse et alvéolée. Elle a trait également à un procédé particulier d'obtention de cette composition et aux utilisations de celle-ci dans l'industrie.

Le 4-O-béta-D-galactopyranosyl-D-glucitol appelé communément lactitol, est un polyol obtenu industriellement par hydrogénation du lactose. Il présente un grand intérêt en raison du fait qu'il est plus stable chimiquement et moins calorique que le saccharose, tout en possédant avantageusement la propriété de pouvoir convenir à l'alimentation des diabétiques. De plus, le lactitol possède la particularité de ne pas être cariogène, ce qui lui ouvre et lui a déjà ouvert, de multiples applications dans l'industrie, notamment dans les industries alimentaires et pharmaceutiques.

Le lactitol est généralement commercialisé sous forme d'une poudre cristalline. Sa cristallographie est particulièrement complexe si l'on se réfère à la littérature, de laquelle il ressort que ce polyol peut cristalliser à la fois sous différentes formes anhydres, monohydratées, dihydratées voire même trihydratées.

Au sujet de la forme anhydre, Wolfrom et al semblent avoir été les premiers en 1938 à faire état dans Journal of the American Chemical Society, 60, 571-573, de l'existence d'un produit cristallin anhydre de point de fusion voisin de 146°C.

Dans la demande de brevet JP 2-200 695 publiée en 1990 sont décrits des cristaux anhydres de lactitol de point de fusion nettement plus faible puisque compris entre 121°C et 124°C.

On connaît aussi un produit cristallisé sous forme monoclinique, de maille élémentaire bien définie, ayant un point de fusion compris entre 149°C et 152°C. Celui-ci a fait l'objet de la demande de brevet WO 92/16542 publiée en 1992. Ce produit ressemble fortement à celui décrit deux années auparavant dans la demande JP 2-255 694.

En ce qui concerne le lactitol cristallisé sous forme monohydrate, c'est-à-dire avec une seule molécule d'eau, Velthuijsen a fait état en 1979 dans Journal of Agriculture, Food and Chemistry, 27, 680-686, d'un produit ayant un point de fusion compris entre 94°C et 97°C. De leur côté, Bommel et al, lors d'une conférence donnée au 6ème Meeting Européen de Cristallographie tenu à Barcelone en 1980, ont présenté les résultats de leurs travaux et ont indiqué qu'en faisant cristalliser du lactitol dans une solution hydroalcoolique à 50 %, on obtenait des cristaux sous forme monohydrate orthorhombique, de maille cristalline particulière et de point de fusion de 102°C.

Toujours concernant la forme monohydratée, la société C.V. CHEMIE COMBINATE AMSTERDAM a rendu publique en 1981 l'existence d'un lactitol monohydrate présentant l'ensemble des caractéristiques décrites par Bommel et al en dehors du fait que le point de fusion du produit indiqué est compris entre 121°C et 123°C. Ce produit a fait l'objet de la demande européenne de brevet EP 039 981.

Enfin, on connaît un produit presque exclusivement constitué par du lactitol monohydrate et ayant un point de fusion se situant entre 90°C et 105°C. Celui-ci présente une maille cristalline élémentaire semblable à celle décrite par Bommel et al et est décrit dans la demande de brevet WO 90/06317 publiée en 1990 et dans la demande de brevet JP 2 196 794 publiée en 1991.

Concernant la forme dihydratée, elle est connue depuis longtemps puisque a priori déjà en 1920 dans une note publiée dans les Comptes Rendus Hebdomadaires des Séances de l'Académie des Sciences, 170, 47-50, J.B. Senderens décrivait des cristaux orthorhombiques de lactitol de point de fusion voisin de 78°C. Les résultats de Wolfrom et al publiés en 1952 dans Journal of the American Chemical Society, 74, 1105, font également état de l'existence d'un lactitol dihydrate de point de fusion compris entre 72,5°C et 74°C. Enfin, Bommel et al lors de la présentation de leurs travaux au 6ème Meeting Européen de Cristallographie de Barcelone en 1980 ont confirmé l'existence de cristaux de lactitol dihydrate de point de fusion proche de 78°C et ont de plus caractérisé la maille cristalline élémentaire de ces cristaux, à la fois dans ses dimensions et sa structure.

Le lactitol peut également cristalliser sous forme trihydrate si l'on se réfère à la demande européenne EP 381 483 publiée en 1990. De tels cristaux ont un point de fusion compris entre 52°C et 56°C. Ceci est confirmé dans la demande japonaise JP 4-13686 publiée en 1992.

On retiendra en définitive que le lactitol peut cristalliser sous différents états d'hydratation et sous des formes plus ou moins stables et qu'il existe généralement pour ce polyol plusieurs formes cristallines pour un même état d'hydratation.

Il faut signaler ici que le nombre de formes cristallines connues du lactitol est sans commune mesure avec celui connu pour d'autres sucres ou polyols. Par exemple on ne connaît à ce jour qu'une seule forme cristalline pour le saccharose, le maltose, le xylitol, l'érythritol ou le maltitol et seulement deux ou trois formes cristallines pour par exemple le dextrose et le mannitol.

Cela explique que dans le cas du lactitol, il a toujours été difficile, contrairement aux autres sucres et polyols, d'obtenir essentiellement une seule forme cristalline non polluée par d'autres formes. A ce jour, la maîtrise de la cristallisation reste imparfaite comme en témoignent en particulier les documents WO 92/16542 et JP 2-255 694 ou encore WO 90/06317 publiés très récemment.

Dans la demande internationale WO 92/16542, il est préconisé pour obtenir des cristaux anhydres de point de fusion voisin de 150°C, de procéder à une cristallisation dans l'eau à partir de solutions sursaturées hautement concentrées, maintenues constamment à température élevée. L'absence d'ajout systématique d'une amorce cristalline destinée à orienter la cristallisation uniquement vers cette forme cristalline, fait que le produit final peut contenir des quantités non négligeables de cristaux de forme hydratée. Aucune autre méthode de fabrication n'est exposée dans ce document pour fabriquer ce produit.

Dans la demande japonaise JP 2-255 694, on retient une autre méthode pour produire des cristaux anhydres de point de fusion voisin de 150°C. On procède selon ce document à une cristallisation en alimentant, de préférence en continu, un mélangeur-extrudeur avec un sirop de lactitol chimiquement pur, concentré à plus de 90 % de matière sèche et maintenu à une température supérieure à 80°C et en amorçant la cristallisation avec des cristaux de lactitol quelconques de façon à ce que celle-ci soit rapide. Il est dit qu'il est impératif de retenir, pour le sirop destiné à être cristallisé, les conditions de concentration et de température rappelées ci-dessus de façon à ne pas contaminer le produit final par des formes hydrates ou une autre forme anhydre que celle recherchée.

Dans cette demande de brevet sont de plus présentées d'autres méthodes envisageables de production de cristaux anhydres comme la cristallisation en solution hydroalcoolique, la cristallisation par la technique dite de "massé" ou encore l'atomisation, mais ces méthodes sont écartées d'office car elles ne permettent pas selon la description, d'obtenir des produits ne contenant que la forme cristalline recherchée de point de fusion proche de 150°C.

Quant à la demande de brevet WO 90/06317 elle a trait à la préparation par cristallisation dans l'eau, dans des conditions de température et de concentration bien définies, de lactitol de façon à n'obtenir que des cristaux sous forme monohydrate de point de fusion particulier. Aucune autre méthode n'est présentée ni même suggérée.

On constate que seules la cristallisation dans l'eau et la cristallisation à l'aide d'un mélangeur-extrudeur sont connues comme pouvant permettre, dans des conditions très particulières, d'obtenir du lactitol cristallisé essentiellement sous une seule forme cristalline. Ces techniques sont d'ailleurs aujourd'hui quasiment les seules à être employées industriellement. Les produits obtenus conviennent particulièrement bien à certaines applications comme celles du chewing-gum ou du chocolat.

Mais, il est d'autres applications où ces produits ne sont pas totalement satisfaisants. C'est le cas par exemple lorsque l'on souhaite utiliser du lactitol pour remplacer le saccharose ou le lactose dans les formes sèches pharmaceutiques telles que les gélules, les médicaments du type poudres à dissoudre, les comprimés et les préparations nutritives pulvérulentes à diluer. C'est également le cas lorsque l'on souhaite réaliser le même genre de substitution dans les aliments sucrés tels que les boissons en poudre, les entremets, les préparations pour gâteaux ou les poudres chocolatées ou vanillées pour petit déjeuner.

On constate pour ces applications particulières, aussi bien pour les poudres cristallines de lactitol obtenues par la technique de malaxage-extrusion que pour les poudres cristallines de lactitol obtenues par cristallisation dans l'eau, que celles-ci présentent plusieurs défauts comme en particulier ceux de s'écouler difficilement, d'être sujettes à une prise en masse ou à un mottage, de ne se dissoudre que très lentement dans l'eau, d'être de mauvais excipients pour compression ou de ne pas satisfaire aux critères d'identification et de pureté imposés par certaines pharmacopées.

La demanderesse a donc cherché à mettre au point une composition cristalline de lactitol anhydre, d'une très grande pureté cristalline, n'ayant pas les défauts d'écoulement, de mottage, de dissolution, ou de compression que présentent les poudres de lactitol connues. Certes, on aurait pu penser que le besoin identifié puisse être satisfait par d'autres polyols. Or, on constate qu'il n'en est rien car aucun d'entre eux ne possède simultanément l'ensemble des caractéristiques métaboliques et physico-chimiques que présente le lactitol.

Il est du mérite de la demanderesse d'avoir réussi, contre toute attente, après avoir mené une recherche approfondie sur le sujet, à préparer une composition cristalline de lactitol anhydre ne présentant pas les défauts relevés pour les poudres de lactitol connues tout en étant d'une grande pureté cristalline. Elle a mis en évidence, de manière surprenante et inattendue, qu'une telle composition cristalline pouvait être préparée dans des conditions particulières à partir d'une solution ou d'une suspension par un procédé s'apparentant à une atomisation, laquelle n'avait jamais permis dans le passé d'obtenir directement des produits de grande pureté cristalline. Il faut noter ici que cela est d'autant plus surprenant que cette technique d'atomisation apparaissait selon la demande de brevet JP 2-255 694, comme étant contre-indiquée pour obtenir un tel résultat.

L'invention a trait par conséquent, en premier lieu, à une composition cristalline de lactitol anhydre, présentant une structure essentiellement poreuse et alvéolée et une pureté cristalline supérieure ou égale à 90 %.

L'invention vise notamment une composition cristalline de lactitol anhydre, présentant une structure essentiellement poreuse et alvéolée, une pureté cristalline supérieure ou égale à 90%, et une densité apparente comprise entre 100 et 620 g/l, de préférence entre 200 et 600 g/l et plus préférentiellement entre 300 et 550 g/l.

La première caractéristique essentielle de la composition de lactitol tient au fait qu'elle est cristallisée essentiellement sous forme anhydre, cet état cristallisé lui conférant une très haute stabilité vis-à-vis de l'humidité. Elle a pour conséquent une faible tendance à prendre en masse ou à motter. Ainsi, son usage est aisé.

Selon une seconde caractéristique essentielle, la composition cristalline de lactitol anhydre selon l'invention présente une pureté cristalline supérieure ou égale à 90 %. La notion de pureté cristalline doit être entendue, dans le cadre de la présente invention, comme correspondant au pourcentage de lactitol cristallisé sous forme anhydre de point de fusion compris entre 145°C et 155°C, exprimé en poids sec par rapport à l'ensemble du lactitol présent dans la composition cristalline. Cette pureté cristalline peut être évaluée aisément par analyse thermique différentielle en considérant qu'un produit complètement cristallisé sous forme anhydre de point de fusion compris entre 145°C et 155°C présente une enthalpie de fusion de l'ordre de 150 joules/g. On calcule alors cette pureté en divisant par 150 joules/g la valeur de l'enthalpie obtenue en creuset hermétique pour la forme anhydre de point de fusion compris entre 145 et 155°C présente dans la composition. De préférence la pureté cristalline de la composition est supérieure ou égale à 95 % et mieux encore supérieure ou égale à 98 %.

La cristallinité de la composition conforme à l'invention est directement proportionnelle à son enthalpie de fusion, laquelle, mesurée en creuset hermétique, est de préférence supérieure à 135 J/g, plus préférentiellement supérieure à 140 J/g et encore plus préférentiellement supérieure à 145 J/g.

Il a été constaté de manière surprenante et inattendue que la composition conforme à l'invention possède une cristallinité en général supérieure ou égale à celle d'un lactitol anhydre de richesse en lactitol équivalente obtenu par malaxage-extrusion. La notion de richesse en lactitol doit être entendue, dans le cadre de la présente invention, dans son sens chimique c'est-à-dire comme correspondant au pourcentage de lactitol exprimé en poids sec/sec par rapport à l'ensemble des carbohydrates présents dans la matière sèche de la composition. Ces carbohydrates peuvent être des sucres comme le lactose ou des polyols comme le mannitol, le sorbitol ou le lactulitol. Habituellement, cette richesse est mesurée par chromatographie liquide haute performance.

C'est ainsi que la composition cristalline de lactitol conforme à l'invention possède de préférence une richesse en lactitol au moins égale à 92 %. Mais on préfère toutefois, afin qu'elle puisse cristalliser directement et plus parfaitement, qu'elle présente une richesse en lactitol supérieure ou égale à 95 %, et mieux encore supérieure ou égale à 98 %. L'idéal est d'atteindre une richesse voisine ou supérieure à 99 %.

Par ailleurs, on préfère également que la composition conforme à l'invention ne contienne qu'une faible teneur en polyols choisis parmi le sorbitol, le mannitol et le lactulitol. La teneur en ces polyols est de préférence inférieure à 5 % et mieux encore inférieure à 2 % par rapport à la matière sèche de la composition. Il a été constaté en effet que leur présence altère de façon significative les propriétés de la composition conforme à l'invention. Ceci n'est pas le cas, ou l'est dans une bien moindre mesure, lorsque la composition contient certaines autres substances. Ceci explique que la composition cristalline de lactitol puisse contenir sans inconvénient de telles substances en quantité plus ou moins grande, en fonction de l'usage qui lui est réservé.

Parmi les substances susceptibles d'entrer dans la composition cristalline de lactitol sans poser de problème majeur, on peut citer par exemple les édulcorants intenses, les colorants, les parfums, les arômes, les vitamines, les minéraux, les principes actifs pharmaceutiques ou vétérinaires, les esters d'acides gras, les acides organiques ou minéraux et leurs sels, les matières protéiques comme les protéines, les acides aminés et les enzymes.

Selon une troisième caractéristique essentielle, la composition cristalline de lactitol conforme à l'invention présente une structure essentiellement poreuse et alvéolée. Au microscope optique on constate qu'elle est essentiellement constituée de particules sphériques sans arêtes vives, ce qui la distingue très nettement de poudres de lactitol obtenues par cristallisation dans l'eau ou par malaxage-extrusion. En microscopie électronique, on constate que la composition conforme à l'invention contient des particules composées de micro-particules agglomérées entre elles. D'ordinaire la composition selon l'invention présente de ce fait une densité aérée plus faible que celles des poudres de lactitol connues. Cette densité peut être mesurée par exemple en utilisant un appareil commercialisé par la société HOSOKAWA sous la marque "Powder Tester" en appliquant la méthode recommandée pour mesurer une densité apparente. Dans ces conditions, pour une coupe granulométrique comprise entre 100 et 200 micromètres (microns), la composition conforme à l'invention présente une densité apparente comprise entre environ 100 et environ 620 g/l, de préférence 200 et 600 g/l et plus préférentiellement entre 300 et 550 g/l. De façon courante, sa densité apparente est comprise entre 350 et 500 g/l.

Il faut noter que la structure essentiellement poreuse et alvéolée de la composition se différencie de façon nette de la structure d'un lactitol cristallisé dans l'eau ou d'un lactitol extrudé, qui sont dans les deux cas constitués de particules cubiques ou parallépipédiques très anguleuses.

C'est ainsi que la composition cristalline de lactitol conforme à l'invention est quasiment dépourvue de particules présentant des caractéristiques de forme et d'aspect similaires à celles retrouvées dans les poudres de lactitol cristallisé dans l'eau ou extrudé.

La composition cristalline conforme à l'invention possède en général une surface spécifique comprise entre 0,2 m²/g et 0,45 m2/g pour une coupe granulométrique de 50 à 200 micromètres (microns). Ces valeurs de surface spécifique sont supérieures à celles obtenues dans le cas d'un lactitol anhydre préparé par cristallisation dans l'eau.

De plus, la demanderesse a constaté, en mesurant la porosité au mercure sur des coupes granulométriques de 100 à 200 micromètres (microns), que la composition conforme à l'invention est constituée de particules possédant des pores ouverts de taille comprise entre 1 et 10 micromètres (microns).

La teneur en eau, déterminée par étuvage à 130°C pendant 2 heures de la composition cristalline de lactitol conforme à l'invention, est de préférence inférieure à 2 % et plus préférentiellement encore inférieure à 1 %. Généralement, cette teneur est même inférieure à 0,5 % voire 0,3 %.

En ce qui concerne les caractéristiques fonctionnelles de la composition cristalline de lactitol conforme à l'invention, la demanderesse a évalué son aptitude à l'écoulement en utilisant l'appareil commercialisé par la société HOSOKAWA. Cet appareil permet de mesurer, dans des conditions standardisées et reproductibles, l'aptitude à l'écoulement d'une poudre et de calculer une note d'écoulement appelée également indice de Carr. La composition conforme à l'invention présente une note d'écoulement excellente, comprise entre 75 et 90. Cette valeur est de préférence comprise entre 77 et 90 et plus préférentiellement entre 80 et 90.

Par ailleurs, l'aptitude à l'écoulement de la composition faisant l'objet de l'invention est d'ordinaire supérieure à celles de poudres de lactitol obtenues par cristallisation dans l'eau.

On peut penser que l'excellente aptitude à l'écoulement de la composition conforme à l'invention s'explique par la combinaison de plusieurs de ses caractéristiques physico-chimiques à savoir en particulier l'absence de charges électrostatiques importantes à la surface des particules la constituant, sa pureté cristalline élevée en lactitol anhydre, sa richesse en lactitol et enfin la forme caractéristique des particules la constituant. Cette excellente aptitude à l'écoulement est avantageuse car elle rend très aisée le remplissage et la vidange de trémis, de récipients ou d'autres contenants tels que sachets ou gélules par exemple.

Une seconde propriété fonctionnelle essentielle de la composition cristalline de lactitol conforme à l'invention, est son aptitude à se dissoudre très rapidement dans l'eau. Pour mesurer cette vitesse de dissolution, on procède selon un test A qui consiste à introduire dans 150 grammes d'eau déminéralisée et dégazée, maintenue à 20°C et soumise à une agitation par barreau magnétique à 200 t/minute dans un bécher de 250 ml de forme basse, 5 grammes exactement d'une coupe granulométrique de 100 et 200 micromètres (microns) du produit à tester. Le temps de dissolution correspond au temps nécessaire après introduction de la coupe granulométrique, pour obtenir une parfaite limpidité visuelle de la préparation. Dans ces conditions, la composition conforme à l'invention possède en général une vitesse de dissolution inférieure à 20 secondes. La composition préférée se dissout en moins de 15 secondes alors que la composition idéale ne nécessite qu'un temps inférieur à 12 secondes. Ces temps sont généralement inférieurs à ceux obtenus avec toutes les poudres de lactitol actuellement commercialisées. On comprend que cette faculté de dissolution rapide soit un avantage indéniable, par exemple dans la fabrication de produits alimentaires ou pharmaceutiques à dissoudre avant leur ingestion.

La composition cristalline de lactitol conforme à l'invention possède également d'autres caractéristiques avantageuses. On peut citer sa très bonne aptitude à être comprimée pour préparer des tablettes à mâcher ou à sucer et sa très bonne aptitude à être mélangée à d'autres produits.

L'invention a trait en second lieu à un procédé de préparation d'une composition cristalline de lactitol anhydre présentant une structure essentiellement poreuse et alvéclée et une pureté cristalline supérieure ou égale à 90 %.

La composition cristalline de lactitol conforme à l'invention est susceptible d'être obtenue en procédant à la pulvérisation d'un sirop relativement riche en lactitol par rapport à la quantité de carbohydrates présents dans ce sirop, sur un lit pulvérulent en mouvement de particules de lactitol cristallisé de richesse au moins égale à celle du sirop. Il a été constaté que la richesse en lactitol du sirop doit être de préférence supérieure ou égale à 92 % afin que la cristallisation du lactitol puisse s'opérer en un temps suffisamment bref et de façon suffisamment importante.

Ce sirop de lactitol est généralement une solution totalement limpide de lactitol. Il peut s'agir aussi d'une suspension légèrement opaque en raison de la présence au sein du sirop de cristaux de lactitol. Dans ce cas, on préfère que ces cristaux soient de très faible taille.

La composition cristalline de lactitol peut en particulier être obtenue en mettant en oeuvre le procédé comportant les étapes suivantes :
- préparation d'un sirop de lactitol ayant une matière sèche d'au moins 50 % et présentant de préférence une richesse en lactitol supérieure ou égale à 92 %,
- pulvérisation fine de ce sirop sur un lit pulvérulent de particules en mouvement de lactitol cristallisé d'une richesse en lactitol de préférence au moins égale à celle du sirop ; la masse du lit représentant constamment au moins 2 fois la masse du sirop pulvérisé,
- séchage du lit pulvérulent et du sirop afin d'obtenir une cristallisation du lactitol sous forme anhydre de point de fusion compris entre 145 et 155°C,
- maturation éventuelle de la composition cristalline de lactitol jusqu'à ce qu'elle présente une cristallinité suffisante et de préférence une enthalpie de fusion supérieure ou égale à 135 J/g,
- recyclage éventuel de la composition cristalline de lactitol après maturation afin qu'elle constitue un nouveau lit pulvérulent de lactitol cristallisé.

Contrairement à ce que l'on aurait pu penser, cette technique permet d'obtenir une composition de lactitol cristallisé à hauteur d'au moins 90 % sous forme anhydre de point de fusion compris entre 145 et 155°C, sans nécessairement retenir pour cela un lit de particules en mouvement de lactitol cristallisé dans cet état anhydre.

Les propriétés de la composition conforme à l'invention peuvent être ajustées en modifiant la richesse en lactitol du sirop, la matière sèche du sirop, la finesse de la pulvérisation, la forme des particules de lactitol cristallisé constituant le lit pulvérulent, le moyen de mise en mouvement de ces particules, la température du lit, la température de séchage, et les masses respectives du lit et de sirop pulvérisé.

En ce qui concerne la richesse en lactitol du sirop, on préfère, sans que cela soit obligatoire, qu'elle soit supérieure ou égale à 95 %, mieux encore supérieure ou égale à 98 %, l'idéal étant de choisir une richesse voisine ou supérieure à 99%.

De préférence, le sirop de lactitol présente une matière sèche comprise entre 55 et 99,5 %. En général cette matière sèche est avantageusement comprise entre 60 et 90 % et mieux entre 65 et 85 %.

Il est préférable d'éviter une pulvérisation grossière du sirop, auquel cas on observe un collage, une mauvaise cristallisation du lactitol et une augmentation très forte de la densité, ce qui n'est pas recherché. Aussi, afin que la composition cristalline de lactitol présente les propriétés spécifiques décrites plus haut, il convient de retenir un matériel permettant de former à partir du sirop de très fines gouttelettes, voire un brouillard.

En ce qui concerne la nature des particules de lactitol constituant le lit pulvérulent, on préfère qu'elles présentent également une grande richesse en lactitol, toujours au moins égale à celle du sirop employé. Pour obtenir un bon résultat, il est aussi préférable que ce lit soit également assez peu dense, c'est-à-dire présente une densité inférieure à 620 g/l et mieux encore inférieure à 600 g/l. L'idéal est de retenir pour ce lit des particules de lactitol présentant l'ensemble des caractéristiques de la composition cristalline de lactitol conforme à l'invention. Ceci peut être obtenu en réalisant un recyclage partiel de la composition conforme à l'invention, laquelle joue alors le rôle de lit pulvérulent de lactitol cristallisé. Il est très avantageux de procéder de la sorte mais alors il est préférable de broyer ou de tamiser la composition conforme à l'invention pour ne retenir que les particules de taille inférieures à 150 micromètres (microns) et mieux encore de taille inférieure à 90 micromètres (microns).

La mise en mouvement des particules constituant le lit pulvérulent peut être obtenue mécaniquement, ou par soufflage d'air. Cette dernière possibilité est préférée car il est facile en choisissant la température de l'air, d'ajuster la température du lit à une valeur comprise entre 60 et 110 °C, et en régulant les débits d'air, d'ajuster les propriétés de la composition cristalline de lactitol.

Généralement, on préfère que la température de ce lit soit maintenue entre 65 et 105 °C; l'idéal étant de se situer entre 70 et 90 °C. On préfère également que la masse du lit pulvérulent représente constamment 3 fois ou mieux 5 fois la masse de sirop pulvérisé. Lorsqu'on procède à un recyclage partiel de la composition selon l'invention afin qu'elle joue le rôle de lit pulvérulent, il suffit d'ajuster le débit d'entrée en sirop pour qu'il ne représente qu'au plus 25 %, ou mieux au plus 17 % du débit d'entrée en composition recyclée.

Le séchage du lit pulvérulent sur lequel a été pulvérisé le sirop doit être conduit de façon à obtenir une teneur en eau finale ne dépassant pas 2 %, de préférence 1 % et plus préférentiellement 0.5 % de la composition.

La demanderesse a démontré que l'on pouvait avantageusement fabriquer en continu la composition cristalline de lactitol, par exemple en utilisant une tour d'atomisation de type M.S.D. de la société NIRO-ATOMIZER laquelle permet, grâce à sa conception, de reproduire toutes les étapes essentielles du procédé conforme à l'invention.

Ce matériel permet, en effet, de pulvériser très finement à l'aide de la buse qu'il comporte, un sirop ayant une température comprise entre 45 et 75 °C et une matière sèche comprise entre 55 et 80 %, sur un lit de particules de lactitol, mis et maintenu en mouvement avec de l'air. De plus, ce matériel permet simultanément d'opérer à un séchage par de l'air sortant de la tour. On peut choisir avantageusement une température d'entrée d'air comprise entre 160 et 280 °C et des débits en matières entrantes tels que la température de l'air sortant de la tour soit comprise entre 60 et 130°C et mieux encore entre 80 et 100°C. Ce matériel permet également de procéder éventuellement à un recyclage partiel de la composition cristalline de lactitol et de la disperser très finement dans le haut de la tour, autour de la buse de pulvérisation du sirop.

La composition cristalline de lactitol obtenue selon le procédé conforme à l'invention peut au besoin être par la suite granulée, de façon à modifier sa granulométrie. Cette granulation peut être réalisée à l'eau, à la vapeur, ou à l'aide d'un sirop contenant de préférence du lactitol.

La composition cristalline de lactitol conforme à l'invention peut avantageusement être employée en tant qu'agent édulcorant, agent de charge ou de texture, excipient ou support d'additifs divers. Elle est particulièrement recommandée, en raison de ses propriétés spécifiques, à la fabrication de comprimés et de poudres à dissoudre dans les domaines alimentaire et pharmaceutique ou autres. Rien n'empêche toutefois de l'utiliser à une tout autre fin, comme par exemple pour formuler des chewing-gums ou des confiseries.

L'invention sera encore mieux comprise à l'aide de l'exemple qui suit, lequel ne se veut pas limitatif et fait seulement état de certains modes de réalisation et de certaines propriétés avantageuses de la composition cristalline de lactitol selon l'invention.

### Exemple : Préparation de compositions cristallines de lactitol selon l'invention et comparaison avec les produits de l'art antérieur.

On prépare une solution de lactitol à 85 % de matière sèche par dissolution de cristaux de lactitol monohydrate présentant une richesse en lactitol de 99,0 %. Cette solution est amenée à 100°C puis maintenue à cette température.

On procède à la pulvérisation de cette solution à l'aide d'une buse sur 100 g d'une poudre de lactitol cristallisé dans l'eau sous forme monohydrate, finement broyée et mise en mouvement dans un appareil de type Aéromatic. On retient pour cela la poudre cristalline LACTY^{R} M commercialisée par la société C.C.A.. Celle-ci joue le rôle de lit pulvérulent de lactitol cristallisé. Cette poudre est mise en mouvement par fluidisation avec de l'air à 80°C.

On continue la pulvérisation fine du sirop à débit constant sur le lit pulvérulent de particules en mouvement de façon à ce qu'au bout d'une demi-heure, la quantité de matière dans l'appareil soit de 900 g. On arrête la pulvérisation et on sèche, pendant 15 minutes, le produit obtenu en le maintenant en mouvement par fluidisation avec de l'air à 80°C. On le refroidit ensuite lentement pour le faire mûrir.

On réitère l'expérience décrite ci-dessus en utilisant en tant que lit pulvérulent de départ le produit mûri obtenu ci-dessus.

Dans ces conditions, on constate que la composition de lactitol finale obtenue à la suite de la seconde expérience est quasiment dépourvue de particules présentant des caractéristiques de forme et d'aspect similaires à celles retrouvées dans la poudre de lactitol monohydrate LACTY^{R} M de départ. En effet, la composition est essentiellement poreuse et alvéolée et est constituée de particules anhydres essentiellement sphériques, dépourvues d'arêtes vives et composées d'une multitude de micro-particules cristallines agglomérées entre elles. Cette composition conforme à l'invention est appelée I. Ses principales caractéristiques sont données dans le tableau ci-après.

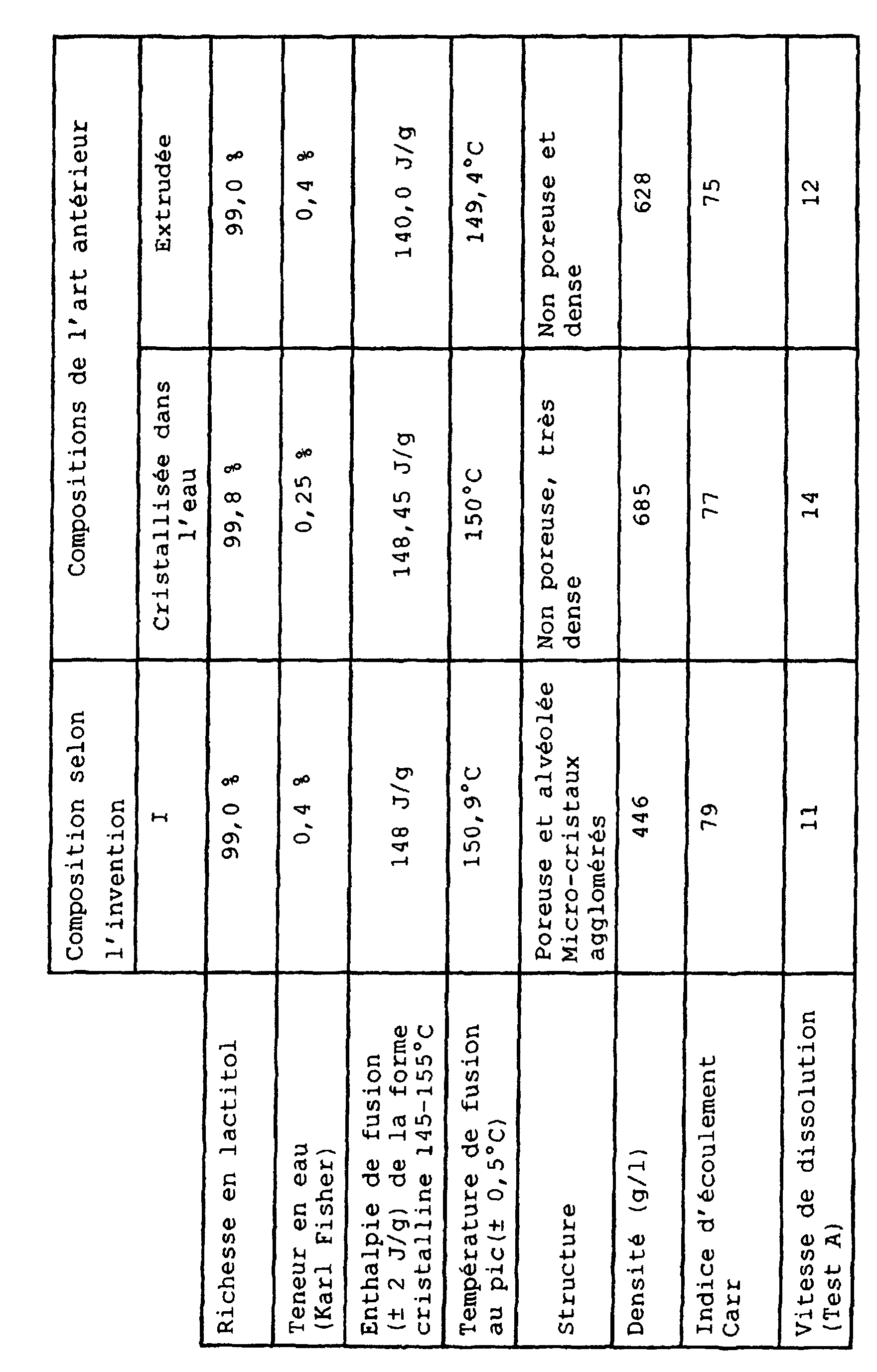

On compare la composition I conforme à l'invention à différentes poudres de lactitol de l'art antérieur, c'est-à-dire :
- une poudre cristalline contenant des cristaux anhydres de lactitol obtenus par cristallisation dans l'eau et commercialisée par la société CULTOR sous le nom LACTITOL AC;
- et une poudre de lactitol extrudée selon les conditions données dans la demande de brevet JP 2-255 694.

La structure des différents produits est observée au microscope optique en lumière polarisée et au microscope électronique, sur des coupes granulométriques de 100 à 200 micromètres (microns). On constate au microscope optique que par comparaison à la poudre cristallisée dans l'eau et à la poudre extrudée, la composition I selon l'invention est essentiellement constituée de particules sphériques sans arêtes vives, ce qui la distingue très nettement des deux autres poudres cristallisées.

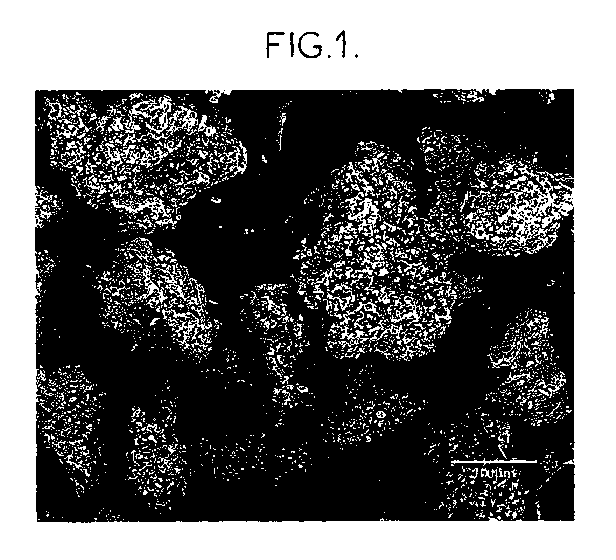

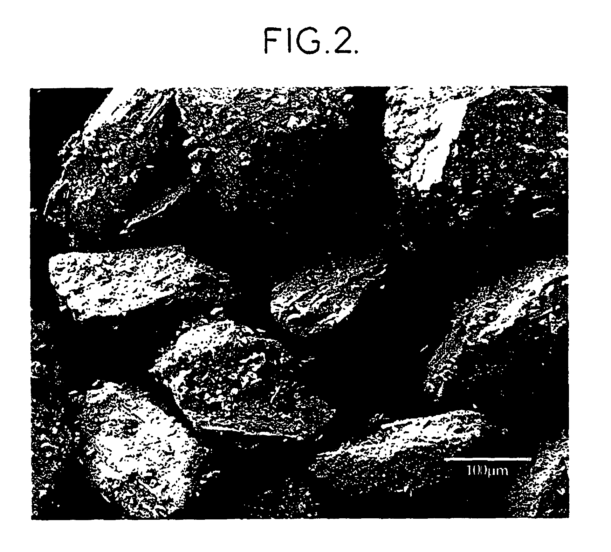

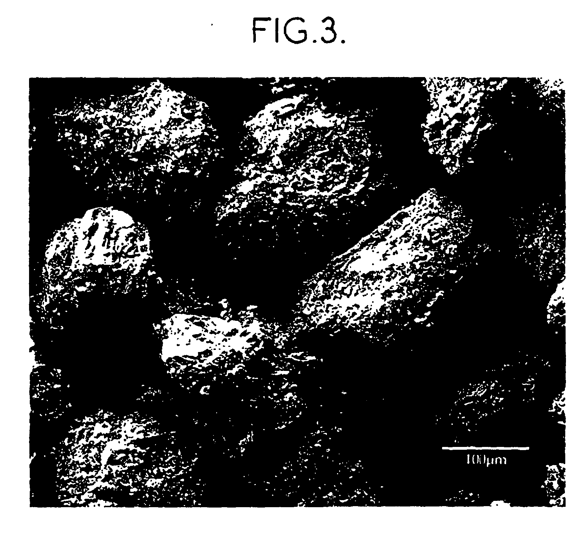

En comparant les photographies obtenues en microscopie électronique pour la composition I (figure 1), la poudre cristallisée dans l'eau (figure 2) et la poudre extrudée (figure 3), on constate que la composition cristalline de lactitol selon l'invention est la seule à posséder essentiellement une structure poreuse et alvéolée et contient des particules composées de micro-particules cristallines agglomérées entre elles. La densité de ces particules apparaît nettement moins élevée que celle des particules des produits de l'art antérieur. Ces dernières présentent en effet une structure dense et compacte, avec des surfaces de particules lisses et des arêtes vives très différentes de celles retrouvées pour la composition selon l'invention.

Dans le tableau suivant sont données plusieurs caractéristiques fonctionnelles des compositions selon l'invention. Contrairement aux compositions de l'art antérieur, les compositions conformes à l'invention allient avantageusement des propriétés jusqu'à présent non retrouvées simultanément. Elles possèdent en effet à la fois les caractéristiques d'être compressibles, de s'écouler facilement et de se dissoudre très rapidement dans l'eau.

De plus il apparaît qu'elles sont très faiblement hygroscopiques, ce qui est un avantage indéniable pour leur stockage et leur usage.

## Revendications

1. Composition cristalline de lactitol anhydre, **caractérisée en ce qu'**elle présente essentiellement une structure poreuse et alvéolée, une pureté cristalline supérieure ou égale à 90 % et une densité apparente comprise entre 100 et 620 g/l, de préférence entre 200 et 600 g/l et plus préférentiellement entre 300 et 550 g/l.

2. Composition selon la revendication 1, **caractérisée en ce qu'**elle présente une richesse en lactitol supérieure ou égale à 92 %, de préférence supérieure ou égale à 95 %, plus préférentiellement encore égale ou supérieure à 98 %, et mieux encore voisine ou supérieure à 99 %.

3. Composition selon l'une ou l'autre des revendications 1 et 2, **caractérisée en ce qu'**elle présente une enthalpie de fusion supérieure à 135 J/g, de préférence supérieure à 140 J/g et plus préférentiellement supérieure à 145 J/g.

4. Composition selon l'une quelconque des revendications 1 à 3, **caractérisée en ce que** sa pureté cristalline est supérieure ou égale à 95 % et mieux encore supérieure ou égale à 98 %.

5. Composition selon l'une quelconque des revendications 1 à 4, **caractérisée en ce qu'**elle contient en poids sur matière sèche, moins de 5 % et de préférence moins de 2% de polyols choisis parmi le sorbitol, le mannitol et la lactulitol.

6. Composition selon l'une quelconque des rendications 1 à 5, **caractérisée en ce qu'**elle présente une note d'écoulement de Carr comprise entre 75 et 90, de préférence comprise entre 77 et 90 et plus préférentiellement comprise entre 80 et 90.

7. Composition selon l'une quelconque des revendications 1 à 6, **caractérisée en ce qu'**elle présente une teneur en eau inférieure à 2 %, de préférence inférieure à 1 % et plus préférentiellement inférieure à 0,5 %.

8. Composition selon l'une quelconque des revendications 1 à 7, **caractérisée en ce qu'**elle contient un ou plusieurs additifs choisis parmi les édulcorants intenses, les colorants, les parfums, les arômes, les vitamines, les minéraux, les principes actifs pharmaceutiques ou vétérinaires, les esters gras d'acides gras, les acides organiques et minéraux et leurs sels, les matières protéiques comme les protéines, les acides aminés et les enzymes.

9. Composition selon l'une quelconque des revendications 1 à 8, **caractérisée en ce qu'**elle présente une vitesse de dissolution dans l'eau selon un test A, inférieure à 20 secondes, de préférence inférieure à 15 secondes et plus préférentiellement inférieure à 12 secondes.

10. Procédé d'obtention d'une composition cristalline de lactitol, **caractérisé en ce qu'**il comporte les étapes suivantes :
- préparation d'un sirop de lactitol ayant une matière sèche d'au moins 50 % et présentant de préférence une richesse en lactitol supérieure ou égale à 92 %,
- pulvérisation fine de ce sirop sur un lit pulvérulent de particules en mouvement de lactitol cristallisé de pureté au moins égale à celle du sirop ; la masse du lit représentant constamment au moins 2 fois la masse du sirop pulvérisé,
- séchage du lit pulvérulent et du sirop afin d'obtenir une cristallisation du lactitol sous forme anhydre de point de fusion compris entre 145 et 155°C,
- maturation éventuelle de la composition cristalline de lactitol jusqu'à ce qu'elle présente une cristallinité suffisante et de préférence une enthalpie de fusion supérieure ou égale à 135 J/g,
- recyclage éventuel de la composition cristalline de lactitol après maturation afin qu'elle constitue un nouveau lit pulvérulent de lactitol cristallisé.

## Patentansprüche

1. Kristalline Zusammensetzung von wasserfreiem Lactitol, **dadurch gekennzeichnet, daß** sie im wesentlichen eine poröse und wabenförmige Struktur, eine kristalline Reinheit von gleich oder größer 90% und ein Schüttvolumen von 100 bis 620 g/l, vorzugsweise von 200. bis 600 g/l und stärker bevorzugt von 300 bis 550 g/l aufweist.

2. Zusammensetzung nach Anspruch 1, **dadurch gekennzeichnet, daß** sie einen Lactitol-Gehalt von größer oder gleich 92%, vorzugsweise von größer oder gleich 95%, stärker bevorzugt von größer oder gleich 98% und noch bevorzugter von größer oder um 99% aufweist.

3. Zusammensetzung nach Anspruch 1 oder 2, **dadurch gekennzeichnet, daß** sie eine Schmelzenthalpie größer 135 J/g, bevorzugt größer 140 J/g und noch bevorzugter größer 145 J/g aufweist.

4. Zusammensetzung nach einem der Ansprüche 1 bis 3, **dadurch gekennzeichnet, daß** ihre kristalline Reinheit größer oder gleich 95% und noch stärker bevorzugt größer oder gleich 98% ist.

5. Zusammensetzung nach einem der Ansprüche 1 bis 4, **dadurch gekennzeichnet, daß** sie, bezogen auf Trockenmasse, weniger als 5% und bevorzugt weniger als 2% unter Sorbitol, Mannitol und Lactulitol ausgewählte Polyole enthält.

6. Zusammensetzung nach einem der Ansprüche 1 bis 5, **dadurch gekennzeichnet, daß** sie einen Carr'schen Rieselfaktor von 75 bis 90, bevorzugt von 77 bis 90 und noch stärker bevorzugt von 80 bis 90 aufweist.

7. Zusammensetzung nach einem der Ansprüche 1 bis 6, **dadurch gekennzeichnet, daß** sie einen Wassergehalt von unter 2%, bevorzugt unter 1% und noch stärker bevorzugt unter 0,5% aufweist.

8. Zusammensetzung nach einem der Ansprüche 1 bis 7, **dadurch gekennzeichnet, daß** sie einen oder mehrere, unter den Süßstoffen, den Färbemitteln, den Parfums, den Aromen, den Vitaminen, den Mineralstoffen, den pharmazeutisch oder tierarzneikundlichen Wirkstoffen, den mit Fettalkoholen veresterten Fettsäuren, den organischen oder mineralischen Säuren sowie deren Salzen, den proteinartigen Substanzen, wie den Proteinen, den Aminosäuren und den Enzymen ausgewählte Zusatzstoffe enthält.

9. Zusammensetzung nach einem der Ansprüche 1 bis 8, **dadurch gekennzeichnet, daß** sie eine mittels eines Tests A bestimmte Lösungsgeschwindigkeit in Wasser von unter 20 Sekunden, vorzugsweise unter 15 Sekunden und stärker bevorzugt unter 12 Sekunden aufweist.

10. Verfahren zur Darstellung einer kristallinen Lactitolzusammensetzung, **dadurch gekennzeichnet, daß** es die folgenden Schritte umfaßt:
- Herstellung eines Lactitolsirups, der einen Anteil an Trockenmasse von mindestens 50% hat und bevorzugt einen Lactitolgehalt von größer oder gleich 92% aufweist,
- Verarbeitung dieses Sirups zu einem feinen Pulver über einem Staubbett aus bewegten kristallisierten Lactitolpartikeln mit einer Reinheit, die wenigstens derjenigen des Sirups gleich kommt, wobei die Masse des Bettes stets mindestens dem Doppelten der Masse des pulverisierten Sirups entspricht,
- Trocknen des Staubbettes und des Sirups, um eine Kristallisation des Lactitols in wasserfreier Form mit einem Schmelzpunkt von 145 bis 155°C zu erhalten,
- gegebenenfalls Reifung der kristallinen Lactitolzusammensetzung, bis diese eine ausreichende Kristallinität und vorzugsweise eine Schmelzenthalpie von größer oder gleich 135 J/g aufweist,
- gegebenenfalls Recyclierung der kristallinen Lactitolzusammensetzung nach der Reifung zur Ausbildung eines neuen Staubbettes aus kristallisiertem Lactitol.

## Claims

1. Anhydrous lactitol crystalline composition, **characterized in that** it essentially exhibits a porous and cellular structure, a crystalline purity greater than or equal to 90% and a bulk density of between 100 and 620 g/l, preferably between 200 and 600 g/l and more preferentially between 300 and 550 g/l.

2. Composition according to Claim 1, **characterized in that** it exhibits a lactitol content greater than or equal to 92%, preferably greater than or equal to 95%, more preferentially still equal to or greater than 98% and better still in the region of or greater than 99%.

3. Composition according to either of Claims 1 and 2, **characterized in that** it exhibits an enthalpy of melting greater than 135 J/g, preferably greater than 140 J/g and more preferentially greater than 145 J/g.

4. Composition according to any one of Claims 1 to 3, **characterized in that** its crystalline purity is greater than or equal to 95% and better still greater than or equal to 98%.

5. Composition according to any one of Claims 1 to 4, **characterized in that** it contains, by weight on a dry basis, less than 5% and preferably less than 2% of polyols chosen from sorbitol, mannitol and lactulitol.

6. Composition according to any one of Claims 1 to 5, **characterized in that** it exhibits a Carr flow grade of between 75 and 90, preferably of between 77 and 90 and more preferentially of between 80 and 90.

7. Composition according to any one of Claims 1 to 6, **characterized in that** it exhibits a water content of less than 2%, preferably of less than 1% and more preferentially of less than 0.5%.

8. Composition according to any one of Claims 1 to 7, **characterized in that** it contains one or more additives chosen from powerful sweeteners, colouring agents, fragrances, flavours, vitamins, minerals, pharmaceutical or veterinary active principles, fatty esters of fatty acids, organic and inorganic acids and their salts, proteinaceous materials, such as proteins, amino acids and enzymes.

9. Composition according to any one of Claims 1 to 8, **characterized in that** it exhibits a rate of dissolution in water according to a test A of less than 20 seconds, preferably of less than 15 seconds and more preferentially of less than 12 seconds.

10. Process for producing a lactitol crystalline composition, **characterized in that** it comprises the following stages:
- preparation of a lactitol syrup having a solids content of at least 50% and preferably exhibiting a lactitol content greater than or equal to 92%,
- fine spraying this syrup onto a pulverulent bed of moving particles of crystalline lactitol with a purity at least equal to that of the syrup; the mass of the bed constantly representing at least twice the mass of the sprayed syrup,
- drying the pulverulent bed and the syrup in order to obtain crystallization of the lactitol in the anhydrous form with a melting point of between 145 and 155°C,
- optional maturation of the lactitol crystalline composition until it exhibits a sufficient crystallinity and preferably an enthalpy of melting greater than or equal to 135 J/g,
- optional recycling of the lactitol crystalline composition after maturation in order for it to constitute a new pulverulent bed of crystalline lactitol.
